Europäisches Patentamt

European Patent Office

Office européen des brevets

(11) Publication number: **0 580 240 A1**

# EUROPEAN PATENT APPLICATION

(21) Application number: 93202118.1

(22) Date of filing: 20.07.93

(51) Int. Cl.⁵: **A23G 3/00**

(30) Priority: **22.07.92 IT MI921772**

(43) Date of publication of application:
**26.01.94 Bulletin 94/04**

(84) Designated Contracting States:
**AT BE CH DE DK ES FR GB GR IE IT LI LU MC
NL PT SE**

(71) Applicant: **ATP AVANT-GARDE
TECHNOLOGIES & PRODUCTS S.A.
MARKETING & LICENSING
5, Via Curti
CH-6900 Lugano(CH)**

(72) Inventor: **Maggi, Giulio Cesare
11, Viale Regina Giovanna
I-20129 Milano(IT)**

(74) Representative: **Riccardi, Sergio
Riccardi & Co.
Via Macedonio Melloni, 32
I-20129 Milano (IT)**

(54) Chewing gum with a varying effect in taste and time.

(57) A chewing gum is described which has a varying effect both regarding its taste and regarding the time during which this taste lasts. Said chewing gum (1) is constituted by a gummy mass (2) having inside a plurality of microcapsules (4,5,6) dispersed in it. Each of said microcapsules contains active nutritional, confectionery and/or medicinal substances, such microcapsules being made with materials able to allow the time necessary for releasing the substances contained in them to be predetermined.

Fig.3

EP 0 580 240 A1

This invention regards a chewing gum with varying effect in time and taste.

Currently numerous ingestable products are known which utilise aromas and, generically, agents which modify taste and odour.

In the particular case of chewing gum the taste-odour is contained in a simple aqueous solution which is immediately released as chewing begins.

This is a disadvantage in that the taste or aroma released remains at very low levels after only a few minutes of chewing, thus quickly losing such particular characteristics and generating disppointment in the consumer who after a short time finds himself chewing a gum with a neutral or even unpleasant taste.

In particular numerous attempts have been made to incorporate pharmaceutical substances in chewing gum, but up till now a chewing gum which releases the active substances incorporated in it in a sustained, controlled and reproducible way has not been able to be obtained given the enormous differences in the ways and forces of mastication which vary from person to person.

The main objective of this invention is that of eliminating the disadvantages stated above in the known types of chewing gum obtaining a chewing gum which allows the intensity of active nutritional, confectionery, and/or medicinal substances contained in them to be kept constant for predetermined times.

Within the scope of the problem described hereinbefore another important objective is that of realising a chewing gum, which adds to the previous characteristics that of releasing in predeterminable temporal succession one or more of said active nutritional, confectionery and/or medicinal products.

Another important objective is that of obtaining a chewing gum which allows the trend of the releasing of said substances to be programmed according to a set temporal curve.

All these problems are particularly serious and difficult when one wishes to incorporate pharmacologically active substances in a chewing gum, obtaining reproducible and reliable performances which are independent of the chewing habits of each person.

Not least is the objective of obtaining a chewing gum which adds to the previous characteristics, that of being pleasant to chew.

The problem and the above mentioned objectives are obtained by a chewing gum with a varying effect in time and taste which is characterised in that it is constituted by a gummy mass with a plurality of microcapsules dispersed in it, said microcapsules containing active alimentary, confectionery and/or medicinal substances and allowing the time necessary for releasing the contents to be predetermined.

Further characteristics and advantages of the invention are shown by the description of particular forms of embodiment given as non-limiting examples, illustrated as an example and not limiting the invention, by the appended sheets of drawings in which:

Figure 1 illustrates a temporal curve of the release of the substances available in a chewing gum of known type;

figure 2 illustrates a perspective view of a chewing gum realised according to the principles of this invention; figure 3 illustrates a sectional view of said gum taken according to a plane of section III-III of figure 2;

figure 4 illustrates a section of an ideal microcapsule;

figures 5 to 7 illustrate temporal curves in relation to particular microcapsules utilised;

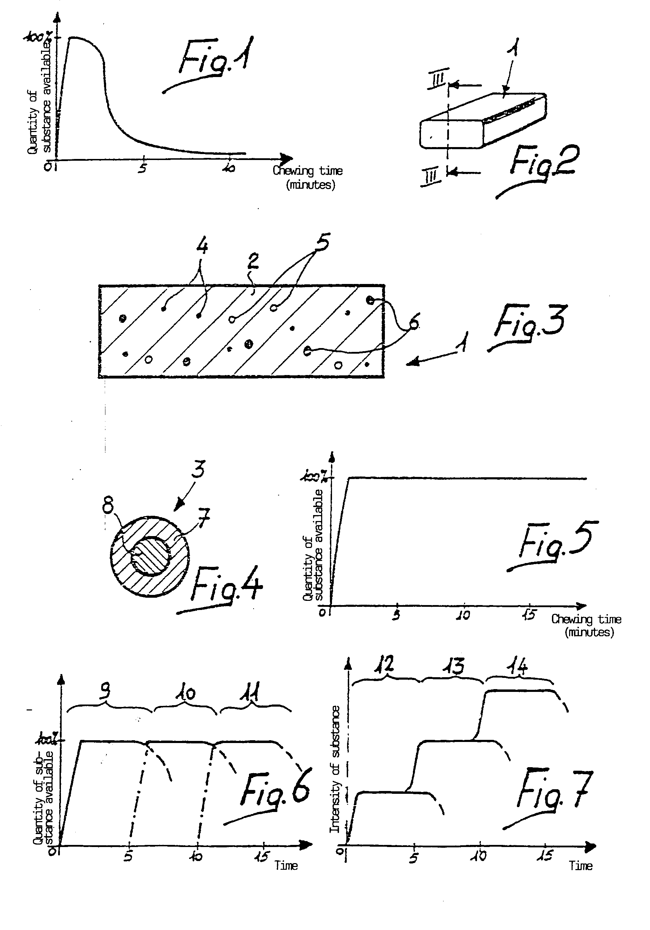

With reference to the figures cited hereinbefore, in Figure 1 the usual condition of temporal decline in the quantity of the substance available in the chewing gum of known type is illustrated.

One can thus verify how the highest quantity of the aroma or taste is immediately released from the first chews, the percentage decreasing to very low values after only a few minutes.

Referring to figures 2, 3 and 4 this invention proposes on the other hand realisation of a chewing gum 1, constituted preferably of a gummy mass 2 which has a plurality of microcapsules 3 inside it which in the particular form of embodiment are emphasised in three different groups, indciated with reference numbers 4, 5 and 6.

Each microcapsule 3 has very limited dimensions, generally less than two tenths of a millimetre, and are constituted by an external layer or membrane 7 of material able to allow it to remain whole in the chewing phase for predetermined times.

On the inside of each microcapsule 3 a core of active substance 8 is present, which may be of nutritional, confectionery and/or medicinal type.

The materials constituting the external layer 7 may be, for example, gelatine, cellulose derivatives, polyvinylpyrrolidone, starch, sucrose, lactose and others in various combinations, as is well known in the techniques of microencapsulation of active substances.

In figure 5 a particular utilisation of the invention is illustrated: it refers to the utilisation of groups of microcapsules containing for example, the same substance 8, which is released in the range of different groups, in successive times and in order to allow availability of the desired substance for the required time which, theoretically, may be indefinite. The variation of release times may be obtained for example by means of membranes 7 of different nature and/or thickness.

In figure 6 a second possible utilisation of the invention is illustrated: in this groups 4, 5, and 6 of microcapsules containing different active substances 8 are considered, the times for the disgregation of the layer 7 remaining unchanged.

During chewing it may therefore be set for a first period 9 of a given substance, for a second period 10 of a different substance for organoleptic or chemical characteristics and so on for a third period 11. Also here, the succession of release time of the substances contained in various groups of microcapsules, may be obtained by means of known technology in the microencapsulation sector.

In figure 7 a further utilisation of the invention is illustrated in which various groups of microcapsules contain the same type of active substance 8, which however is present in increasing quantities, the external layer or membrane 7 allowing the substance to be availabile according to predeterminable times by varying its composition and/or thickness.

One may thus for example arrange for a first period 12 of a given quantity of substance for a set time, a greater quantity in a second period 13, again for a predeterminable time, and lastly in a subsequent period 14 for a further increase of available quantity again for a predeterminable time.

It can therefore be seen how the invention has reached the established aim and objectives, it being possible to predetermine the quantity of active substance available during chewing both with regard to its quantity and its duration with respect to time.

As an example, this active substance may be constituted either by a element for modifying taste or aroma, or by a pharmaceutical substance for example for oral hygiene treatment and for bringing flouride to the dental apparatus. Naturally one may use as the active substance any pharmaceutically active substance which can be administered orally, and in particular those pharmaceutical substances and/or medicines which it has already been attempted to include in chewing gum of a conventional type, such as for example dimenhydrinate, paracetamol, vitamin C, cimetidine, sucralphate, D-metrofane, ginseng and so on.

It has also been demonstated how the possibility has been achieved of obtaining, for example, a taste which is constant for even prolonged chewing times or a taste which increases in intensity with time or again the possibility of arranging for different tastes for successive chewing times.

The size of the microcapsules are such as not to be noticed by the tongue during chewing thus avoiding any unpleasant sensation during the same.

Another noteworthy advantage of this invention is that this chewing gum can be made at cold temperatures, directly pressing the formula in the relevant moulds, using simple equipment already used normally for such production, and thus without any necessity for employing special methods or machinery.

Practical experiments have been carried out of incorporation in the chewing gum of two pharmaceutical substances which are particularly interesting for administration in this galenic form, that is dimenhydrinate and vitamin C. The results are reported in the following examples which must however be considered as purely illustrative and not limiting the scope of the invention.

Preliminarily to the in vivo experiments, analyses were carried out of the release in vitro of the chewing gum containing respectively dimenhyrinate and vitamin C, subsequently administered to volunteers taking part in the experiments, obtaining the following results.

| DIMENHYDRINATE 25 mg | | | |
|---|---|---|---|
| | Tablets Lot P/002 | Tablets Lot P/003 | Tablets Lot 100/ME |
| Release time | % release | % release | % release |
| 3 min. | 75.97 % | 66.55 % | 37.30 % |
| 6 | 84.54 % | 74.47 % | 43.70 % |
| 10 | 87.39 % | 79.15 % | 48.30 % |
| 15 | 88.93 % | 79.15 % | 51.80 % |
| 25 | 88.93 % | 84.91 % | 54.70 % |
| 30 | 89.15 % | 87.43 % | 55.85 % |

| VITAMIN C (L-ascorbic acid) | | |
|---|---|---|
| | Tablets Lot P/002 (250 mg) | Tablets Lot P/002 (500 mg) |
| Release time | % release | % release |
| 7 min. | 90.70 % | 95.50 % |
| 14 | 96.64 % | 98.44 % |
| 21 | 97.58 % | 101.44 % |

EXAMPLE 1

BIOAVAILABILITY OF DIMENHYDRINATE IN A CHEWABLE FORM IN HEALTHY VOLUNTEERS

Dimenhydrinate, a combination of diphenhydramine and 8-chloro-theophylline in equal molecular proportions, a representative HI antagonist, is presented in a special excipient: the drug was administered orally, in a chewable form, to 6 human volunteers of male sex, at the single dose of 2 x 25 mg (total amount 50 mg), in an open design, to evaluate the relative bioavailability.

The investigation was carried out with the aim of ascertaining if the active principle is absorbed from the chewable dosage form. The average weight of each chewing gum was 1500 mg, with mint flavour, containing, 25 mg of active substance.

As a very preliminary procedure, the bioavailability was determined in terms of presence or not of theophylline in the blood (plasma), considering the fact that dimenhydrinate is a salt of diphenydramine containing 50% of theophylline.

The volunteers were active, ambulatory adults, of male sex, with no evidence of diseases and had taken no medicines at least 7 days before entering the study. Blood chemical analysis (Hb, RBC, WBC, PTS, Glucose, Creatinine, urine analysis) were performed 2 - 3 days before the trial and were in the normal range.

Each subject fasted overnight prior to drug treatment: no coffee, tea, or chocolate was eaten the day prior to the trial.

Each volunteer at 08:00 chewed at the same time two tablets of dimenhydrinate (50 mg of active principle) for 5 minutes.

Two hours after the administration, a fruit juice was given to the volunteers.

Venous blood samples were withdrawn with heparinized syringes just before (blanc) and 0.5, 1, 2, 3 and 4 hours after administration of the drug.

Samples were centrifuged at 3000 rpm for 10 minutes and the plasma was separated and frozen (-20°C) until the assay.

Due to the small number of subjects no statistical evaluation of data was done.

To measure the blood (plasma) concentration in theophylline an analytical immunometric procedure was used.

The method is specific and the lowest detectable concentration is <1 $\mu$g/mL of plasma (Immunometric Ektachrom Kodak (dry/method) ). The sensibility is linear between 1 - 40 $\mu$g.

Table 1 shows plama concentration ($\mu$g/mL or the absence (N) of theophylline after administration of dimenhydrinate to the volunteers.

Table 1

| Concentration of theophylline in plasma ($\mu$g/mL) of volunteers treated with 50 mg (2 x 25 mg) Dimenhydrinate | | | | | | | | |
|---|---|---|---|---|---|---|---|---|
| Case | Sex | years | time 0 (blanc) | 0,5 | 1 | 2 | 3 | 4 |
| 1. B.G. | M | 32 | N | 2,3 | 5,1 | 6,0 | 4,6 | 3,2 |
| 2. S.M. | M | 27 | N | 0,9 | 3,0 | 2,9 | 3,1 | 3,0 |
| 3. C.L. | M | 29 | N | 1,4 | 2,4 | 3,2 | 3,0 | 2,8 |
| 4. M.F. | M | 44 | N | 1,2 | 2,1 | 1,9 | 2,2 | 1,8 |
| 5. C.D. | M | 35 | N | 1,5 | 3,0 | 2,8 | 2,9 | 3,1 |
| 6. G.S. | M | 41 | N | 2,0 | 4,1 | 4,8 | 3,7 | 2,9 |

One volunteer (No. 2) 1.5 hours after the administration of the drug felt very slight symptoms of fatigue, which in any case disappeared spontaneously 1 hour later. (A slight local (buccal) anaesthetic effect was present in all volunteers).

Preliminary data obtained in this investigation lead to conclude that dimenhydrinate in the chewable form according to this Example is absorbed, considering only the plasma levels of theophylline, present in the molecule of dimenhydrinate in equal molecular proportions to diphenhydramine, as a "marker" of the drug.

The results of these investigations clearly demonstrate that the performances of the chewable tablet according to the invention are unexpected as the previous attempts to incorporate dimenhydrinate in a chewing gum failed to give a sustained release and a pharmacologically active concentration of the drug in the plasma.

EXAMPLE 2

CHEWING GUM WITH VITAMIN C 250 MG AND 500 MG

A pilot study of bioavailability of a chewing gum containing vitamin C as active substance was conducted on 6 healthy adult volunteers in order to assess the pharmacokinetic behaviour of the 500 mg active principle formulation.

The volunteers were aged between 22 - 43 years, on a normal standard diet. A preliminary clinical examination including ECG and blood, hepatic and renal functions, was performed.

Each subject fasted overnight prior to the administration of vitamin C (500 mg) in chewing gum formulation according to the invention: the study design was open. The blood levels at 0.5-1-2-3-4-6 hours and the cumulative urinary excretion (0 to 6 hours) were estimated, by means of the classical method of 2,4 - dinitrophenylhydrazine. Due to the small number of treated subjects no statistical analysis was performed in the study.

No side effects were recorded.

The results of individual plasma levels (ascorbic + dihydroascorbic acid) during the 5 hours of observation, reported in the following Table 2, enable to point out that:

1) ascorbic acid of the above mentioned formulation is rapidly absorbed (0.5 - 1 hour);
2) the plasma levels at 2-3-5 hours are in the "therapeutic range";
3) no side effects (general and local) were reported during the period of study.

Subclinical deficiency of Vitamin C is very common today (elderly people, nursing mothers, smokers, women under oral contraceptive treatment and people with bad alimentary habits): so a daily supplement of 250 - 500 mg of Vitamin C is mandatory to improve the quality of life and increases the immunological defences.

This test also demonstrates the unexpected performance of the chewable tablet, attaining long standing plasma levels of Vitamin C in the therapeutic range.

TABLE 2. PLASMA CONCENTRATION OF ASCORBIC, DEHYDROASCORBIC ACID IN 6 HEALTHY VOLUNTEERS ON FREE DIET. (AS µmol/L) and CUMULATIVE URINARY EXCRETION (mg from 0800 to 1300)

| Vol No | SEX | AGE (years) | WEIGHT (kg) | Basal | 0,5 H | 1 H | 2 H | 3 H | 5 H | URINE (recovery) |
|---|---|---|---|---|---|---|---|---|---|---|
| 1 | M | 34 | 68 | 22 | 30 | 120 | 182 | 196 | 190 | 176 |
| 2 | M | 36 | 72 | 36 | 51 | 96 | 144 | 158 | 164 | 144 |
| 3 | M | 22 | 70 | 48 | 63 | 130 | 138 | 172 | 150 | 266 |
| 4 | M | 29 | 80 | 62 | 50 | 143 | 206 | 225 | 192 | 190 |
| 5 | F | 40 | 65 | 54 | 69 | 116 | 190 | 210 | 190 | 310 |
| 6 | F | 43 | 68 | 41 | 82 | 109 | 189 | 262 | 136 | 110 |

Figures are the mean of 2 consecutive analysis.

Naturally the invention thus conceived is susceptible to numerous modifications, and variations, all coming within the scope of the same inventive concept.

Thus the inventive number of microcapsules present inside the chewing gum may be any, as may the number of microcapsules belonging to the same group.

Furthermore the type of substances used as well as their quantity, both inside each microcapsule and in the range of each group, may be any, according to requirements.

EP 0 580 240 A1

**Claims**

1.  Chewing gum with varying effect in taste and time, characterized in that it is constituted by a gummy mass with a plurality of microcapsules containing active nutritional, confectionery and/or medicinal substances dispersed in it, said microcapsules allowing the time necessary for releasing their contents to be predetermined.

2.  Chewing gum according to claim 1, characterised in that the microcapsules have an external layer realised with materials able to maintain its integrity in the chewing phase for a predeterminable time.

3.  Chewing gum according to claims 1 and 2, characterised in that the microcapsules have a core of active substances inside them which are utilised in food sector, the confectionery sector or the pharmaceutical sector, said substances being present in predeterminable quantities and/or having different organoleptic characteristics.

4.  Chewing gum according to the preceding claims characterised in that inside the gummy mass is one or more groups of microcapsules having different external layers and/or different active substances inside, the latter in a predeterminable quantity.

5.  Chewing gum according to the preceding claims, characterized in that the external layer of the microcapsules is preferably constituted by gelatine and/or cellulose derivatives and/or polyvinylpyrrolidone and/or starch and/or sucrose and/or lactose in various combinations, the thickness of the external layer being variable according to the desired duration of the time in which the layer must remain whole before beginning to release the active substance contained in the microcapsule.

6.  Chewing gum according to the preceding claims, characterized in that the microcapsules have dimensions preferably less than two tenths of a millimetre.

7.  Chewing gum according to one or more of the preceding claims, characterised in that the microencapsulated active substance is a sustained release pharmaceutically active substance.

8.  Chewing gum according to claim 7, characterised in that the pharmaceutically active substance is dimenhydrinate.

9.  Chewing gum according to claim 8, characterised in that the dimenhydrinate is incorporated in doses of 25 mg of active substance for each chewable tablet.

10. Chewing gum according to claim 7, characterised in that the pharmaceutically active substance is vitamin C in doses of 250 - 500 mg per chewable tablet.

7

Fig.1

Fig.2

Fig.3

Fig.4

Fig.5

Fig.6

Fig.7

## DOCUMENTS CONSIDERED TO BE RELEVANT

| Category | Citation of document with indication, where appropriate, of relevant passages | Relevant to claim | CLASSIFICATION OF THE APPLICATION (Int.CL5) |
|---|---|---|---|
| X | EP-A-0 278 994 (ALFA GUM INVESTMENT INC.) * the whole document * | 1-7 | A23G3/30 |
| Y | | 8-10 | |
| Y | EP-A-0 371 584 (WARNER LAMBERT) * page 5, line 30 - line 39 * * page 5, line 55 - line 58 * * page 6, line 25 - line 29 * | 8,9 | |
| X | EP-A-0 217 109 (R. CAPELLARI) * the whole document * | 1-7 | |
| X | US-A-4 386 106 (C.G. MERRIT ET AL.) * column 7, line 48 - column 8, line 13; claims 1,16,17,19,22 * | 1-6 | |
| Y | EP-A-0 372 695 (WARNER LAMBERT) * page 4, line 37 - line 51 * * page 5, line 1 - line 8 * | 10 | |

TECHNICAL FIELDS SEARCHED (Int.Cl.5)

A23G

The present search report has been drawn up for all claims

| Place of search | Date of completion of the search | Examiner |
|---|---|---|
| THE HAGUE | 27 October 1993 | LEPRETRE, F |

EPO FORM 1503 03.82 (P04C01)